# EUROPEAN PATENT APPLICATION

(11) **EP 3 945 483 A1**
(43) Date of publication of application: **02.02.2022**
(21) Application number: 21187964.8
(22) Date of filing: 27.07.2021
(51) Int. Cl.: G06Q 10/08, G06Q 10/02, G06Q 10/06, G16H 40/00, G16H 50/00, G16H 10/40, G16H 40/20, G16H 50/20

(54) **TRANSPORTATION MANAGEMENT SYSTEM, TRANSPORTATION MANAGEMENT METHOD, AND PROGRAM**

(30) Priority: 28.07.2020 JP 2020127648
(71) Applicant: TOYOTA JIDOSHA KABUSHIKI KAISHA, Toyota-shi, Aichi-ken 471-8571 (JP)
(72) Inventor: ITOZAWA, Yuta, Aichi-ken, 471-8571 (JP); IWAMOTO, Kunihiro, Aichi-ken, 471-8571 (JP); KOMURA, Hirotaka, Aichi-ken, 471-8571 (JP)
(74) Representative: D Young & Co LLP

(57) **Abstract**

A transportation management system according to an aspect is a transportation management system configured to manage transportation of a testing apparatus (50) for diagnosing a health condition of a user by using a computer. A reservation for a test that a user undergoes is received, and a testing apparatus (50) to be transported to the user is determined based on the reservation. The testing apparatus (50) is transported to the user, and the user is diagnosed by using the testing apparatus (50).

## Description

### BACKGROUND

The present disclosure relates to a transportation management system, a transportation management method, and a program.

Japanese Unexamined Patent Application Publication No. 2002-007568 discloses a system in which a user takes an image of a predetermined part of his/her body in his/her house by using an image pickup apparatus, and a server that has received the taken image through a network diagnoses the health condition of the user.

### SUMMARY

In the system disclosed in Japanese Unexamined Patent Application Publication No. 2002-007568, there is a problem that a diagnosis can be carried out based only on an image(s) taken by the image pickup apparatus, and a test using any testing apparatus other than the image pickup apparatus cannot be carried out. Further, there is another problem that it is difficult for a user to have a testing apparatus(es) other than the image pickup apparatus in his/her house because of its cost.

The present disclosure has been made in view of the above-described circumstances, and an object thereof is to provide a transportation management system that manages transportation of a testing apparatus so that a test is carried out at a low cost.

A first exemplary aspect is a transportation management system configured to manage transportation of a testing apparatus for diagnosing a health condition of a user by using a computer, the transportation management system being further configured to:
receive a reservation for a test that a user undergoes;
determine a testing apparatus to be transported to the user based on the reservation; and
transport the testing apparatus to the user and diagnose the user by using the transported testing apparatus.

Further, another exemplary aspect is a transportation management method for managing transportation of a testing apparatus for diagnosing a health condition of a user by using a computer, including:
receiving a reservation for a test that a user undergoes;
determining a testing apparatus to be transported to the user based on the reservation; and
transporting the testing apparatus to the user and diagnosing the user by using the transported testing apparatus.

Further, another exemplary aspect is a program for managing transportation of a testing apparatus for diagnosing a health condition of a user by using a computer, the program being adapted to cause the computer to:
receive a reservation for a test that a user undergoes;
determine a testing apparatus to be transported to the user based on the reservation; and
transport the testing apparatus to the user and diagnose the user by using the transported testing apparatus.

As described above, in an aspect of the present disclosure, a testing apparatus is transported to a user and the user is diagnosed by using the transported testing apparatus. Therefore, a user does not need to have a testing apparatus(es) in his/her house, so that a test can be carried out at a low cost.

The testing apparatus may be transported together with an article that the user has ordered, and the user may be diagnosed by using the testing apparatus when the user receives the article. By the above-described configuration, the test can be carried out at a lower cost than the cost that is required when only the testing apparatus is transported.

The testing apparatus may be put into and transported by an autonomous traveling vehicle. By the above-described configuration, the test can be carried out at a low cost.

After the user is diagnosed by using the testing apparatus, a result of the test of the user may be deleted from the testing apparatus. By the above-described configuration, the result of the test, which is personal information, can be prevented from being leaked.

The user may be referred to a hospital based on the result of the test obtained by diagnosing the user by using the testing apparatus. By the above-described configuration, the time and effort that the user takes to find a hospital can be reduced.

According to the present disclosure, it is possible to provide a transportation management system that manages transportation of a testing apparatus so that a test can be carried out at a low cost.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is a block diagram of a transportation management system according to a first embodiment;
Fig. 2 is a flowchart showing a transportation management method according to the first embodiment;
Fig. 3 is a block diagram of a transportation management system according to a second embodiment; and
Fig. 4 is a block diagram of a transportation management system according to a third embodiment.

### DESCRIPTION OF EMBODIMENTS

Specific embodiments will be described hereinafter in detail with reference to the drawings. The same or corresponding elements are denoted by the same reference numerals (or symbols) throughout the drawings, and redundant descriptions thereof are omitted as required for clarifying the explanation.

### (First Embodiment)

### <Configuration of Transportation Management System>

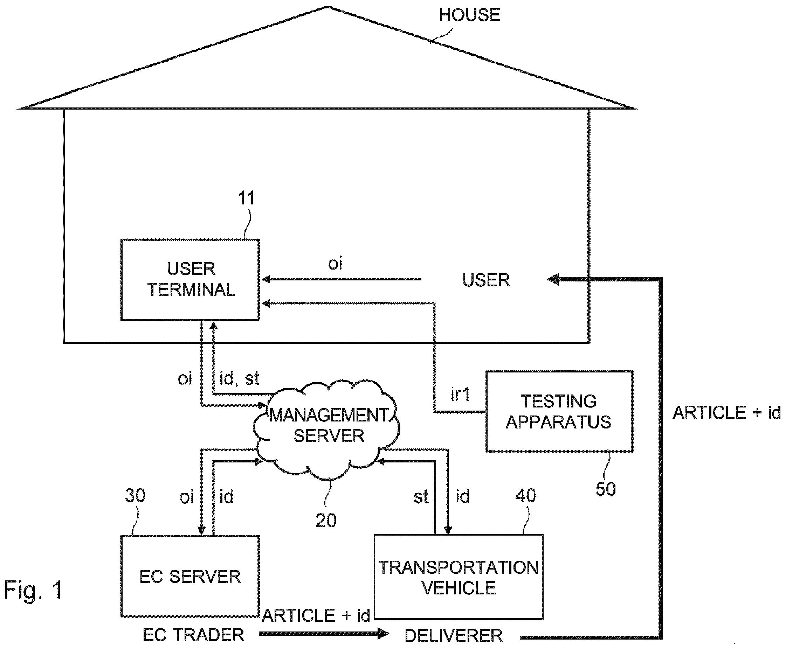

Firstly, a transportation management system according to a first embodiment will be described with reference to Fig. 1. Fig. 1 is a block diagram of a transportation management system according to the first embodiment. As shown in Fig. 1, the transportation management system according to this embodiment includes a user terminal 11, a management server 20, an EC (Electronic Commerce) server 30, a transportation vehicle 40, and a testing apparatus 50.

In the transportation management system according to this embodiment, the transportation of the testing apparatus 50 for diagnosing the health condition of a user is managed by using a computer. The transportation vehicle 40 transports the testing apparatus 50 together with an article(s), and the user is diagnosed by using the testing apparatus 50 when the user receives the article(s). That is, the transportation management system for the testing apparatus according to this embodiment also serves as a delivery management system for articles.

Each of the user terminal 11, the management server 20, the EC server 30, the transportation vehicle 40, and the testing apparatus 50 includes, for example, an arithmetic unit such as a CPU (Central Processing Unit), and a storage unit such as a RAM (Random Access Memory) and a ROM (Read Only Memory) in which various control programs, data, and the like are stored. That is, each of the user terminal 11, the management server 20, the EC server 30, the transportation vehicle 40, and the testing apparatus 50 has a function as a computer, and they perform the below-described processes based on the aforementioned various control programs.

As shown in Fig. 1, the user terminal 11 is a communication terminal that is connected to the management server 20 through a wire or wirelessly so as to be able to communicate with the management server 20. The user terminal 11 is not limited to any particular apparatuses and the like as long as it can communicate with the management server 20, and may be, for example, a mobile communication terminal such as a smartphone or a tablet-type terminal, or a PC (Personal Computer) disposed indoors.

Note that although the user terminal 11 is located in a living space of a house in Fig. 1, it may be located outside the house, or outside the living space in the house. Further, in this specification, the living space includes an office space.

As shown in Fig. 1, the user enters order information oi into the user terminal 11. The order information oi is then transmitted from the user terminal 11 to the management server 20. The order information oi is information about an article(s) to be delivered, and includes, for example, information such as a name of the article, a quantity, a desired delivery date and time, a delivery destination (an address), and the like.

Further, in the transportation management system according to this embodiment, the order information oi includes a reservation for a test that the user undergoes when the user receives the article. Note that the desired delivery date and time included in the order information oi corresponds to a desired test date and time, and the delivery destination (the address) corresponds to a testing place.

Note that the user may only make a reservation for a test without ordering any article. In such a case, the order information oi includes no information about the order of an article, and includes only the information about the reservation for the test (such as a type of the test, a desired date and time of the test, and a place of the test).

The management server 20 manages the delivery of the article and the transportation of the testing apparatus 50. As shown in Fig. 1, the management server 20 is connected to the user terminal 11, the EC server 30, and the transportation vehicle 40 so as to be able to communicate with them. The management server 20 is, for example, a cloud server. The management server 20 transmits the order information oi received from the user terminal 11 to the EC server 30.

Further, the management server 20 determines a testing apparatus to be transported to the user based on the reservation for the test included in the order information oi. For example, a plurality of testing apparatuses are registered for each type of tests in the management server 20. When a reservation for a test is included in the order information oi, the management server 20 determines a type of a testing apparatus based on the type of the test, and determines a testing apparatus to be transported based on the information such as the desired delivery date and time and the delivery destination (the address) included in the order information oi.

Further, the management server 20 also receives order identification information id issued by the EC server 30 and transmits the received order identification information id to the user terminal 11 and the transportation vehicle 40. The order identification information id is information for identifying the article to be delivered, and is, for example, an order number thereof. The testing apparatus to be transported is also associated with the order identification information id.

Further, the management server 20 receives status information st indicating a delivery status of the article from the transportation vehicle 40 and transmits the received status information st to the user terminal 11. The status information st includes, for example, information such as "Waiting to be delivered", "In-delivering" or "Delivered" as the delivery status of the article.

The EC server 30 is, for example, a server constituting an EC site on the Internet managed by an EC trader. As shown in Fig. 1, the EC server 30, which is connected to the management server 20 so as to be able to communicate with the management server 20, issues order identification information id according to the order information oi received from the management server 20 and transmits the issued order identification information id to the management server 20.

Note that instead of the management server 20, the EC server 30 may determine the testing apparatus to be transported to the user based on the reservation for the test included in the order information oi.

The transportation vehicle 40 is a vehicle that transports the testing apparatus 50 as well as delivering the article. The article and the testing apparatus 50 may be put into the transportation vehicle 40 at locations different from each other, or may be put into the transportation vehicle 40 together at the same location. The transportation vehicle 40 is, for example, an autonomous traveling vehicle. The test can be carried out at a low cost by using an autonomous traveling vehicle.

Further, the transportation vehicle 40 includes, for example, a reader capable of reading the order identification information id attached to the article. As shown in Fig. 1, the transportation vehicle 40 is connected to the management server 20 so as to be able to communicate with the transportation vehicle 40. The transportation vehicle 40 receives the order identification information id from the management server 20, and transmits the status information st indicating the delivery status of the article (i.e., the transportation status of the testing apparatus 50) to the management server 20.

In the example shown in Fig. 1, a deliverer (i.e., a person or the like who delivers the article) is on the transportation vehicle 40, so that the deliverer gets off the transportation vehicle 40 and delivers the article to the user. However, the transportation vehicle 40 may be equipped with a delivery robot (including an autonomous traveling vehicle) instead of being operated by the deliverer, and this delivery robot may get off the transportation vehicle 40 and delivers the article to the user.

The order identification information id is, for example, text (i.e., letters), a symbol, a bar code, a 2D (two-dimensional) code, a RFID (Radio Frequency IDentifier), or the like, and is directly or indirectly assigned to the article. More specifically, the order identification information id is directly stuck on the article or embedded in the article. Alternatively, the order identification information id may be stuck on or embedded in the package of the article, and may be indirectly assigned to the article. Note that the package is not limited to any particular packages, and may be, for example, a box, a bag, or a sheet made of paper or vinyl. The package may be a disposable type, or may be a reusable type such as a returnable box.

Note that as shown in Fig. 1, the article to which the order identification information id is attached is delivered from the EC trader to the user by the transportation vehicle 40.

For example, the status information st, which indicates the delivery status of the article, is "Waiting to be delivered" in a period from when the transportation vehicle 40 receives the order identification information id to when the deliverer receives the article from the EC trader.

When the deliverer receives the article from the EC trader and reads the order identification information id assigned to the article by using the reader of the transportation vehicle 40, the status information st changes to "In-delivering".

Then, when the deliverer has delivered the article to the user (i.e., when the delivery of the article has been completed) and the order identification information id assigned to the article is read again by the reader of the transportation vehicle 40, the status information st changes to "Delivered". Note that the reader of the transportation vehicle 40 is, for example, one that can perform wireless communication and is portable, so that the deliverer can carry it with him/her when he/she delivers the article.

Note that the status information st is not indispensable. On the other hand, the status information st may also be transmitted to the EC server 30.

The testing apparatus 50 is a testing apparatus for performing a test that the user undergoes when he/she receives the article. The testing apparatus 50 may notify the user of an abnormality in the result ir1 of the test (hereinafter referred to as the test result ir1), and when there is an abnormality in the test result ir1, the testing apparatus 50 may ask the user a question(s) about the contents of the test result ir1. Further, the testing apparatus 50 may diagnose the health condition of the user based on the test result ir1 and the answer(s) to the question(s).

The testing apparatus 50 is transported by the transportation vehicle 40 together with the article ordered by the user. Then, the deliverer unloads (or takes out) the testing apparatus 50 from the transportation vehicle 40 and delivers it to the user. In the example shown in Fig. 1, the testing apparatus 50 is connected to the user terminal 11 so as to communicate with the user terminal 11, and transmits the test result ir1 to the user terminal 11. Since the test result ir1 is personal information, the test result ir1 may be deleted from the testing apparatus 50 after the test result ir1 is transmitted to the user terminal 11. There is no restriction on the payment for the cost of the test, and for example, electronic money is used.

Note that instead of delivering the testing apparatus 50 to the user, the user may get on the transportation vehicle 40, which has transported the testing apparatus 50, and the user is diagnosed by the testing apparatus 50 carried in the transportation vehicle 40. Further, instead of transmitting the test result ir1 to the user terminal 11, the test result ir1 may be displayed in a display unit of the testing apparatus 50. Further, the testing apparatus 50 may be connected to the management server 20 and/or the transportation vehicle 40 so as to be able to communicate with them. The management server 20, instead of the testing apparatus 50, may diagnose the health condition of the user based on the test result ir1.

Examples of the test performed by using the testing apparatus 50 include: tests for obtaining electrical information through electrodes such as electrocardiography, electroencephalography, and electromyography; and biological tests such as a pulmonary function test, ultrasonography, phonocardiography, a basal metabolism test, and a thermography test. Further, examples of the test performed by using the testing apparatus 50 may also include a specimen test such as an X-ray test, a bone-density test, a urinalysis using a specimen collected from the user, a fecal test, and a blood test.

As described above, in the transportation management system according to this embodiment, the testing apparatus 50 is transported to a user, and the user is diagnosed by using the testing apparatus 50. Therefore, in the transportation management system according to this embodiment, a user does not need to have a testing apparatus(es) in his/her house, so that a test can be carried out at a low cost. Further, in the transportation management system according to this embodiment, since the testing apparatus 50 is transported together with an article(s) ordered by the user, the test can be carried out at a lower cost than the cost that is required when only the testing apparatus 50 is transported.

### transportation Management Method>

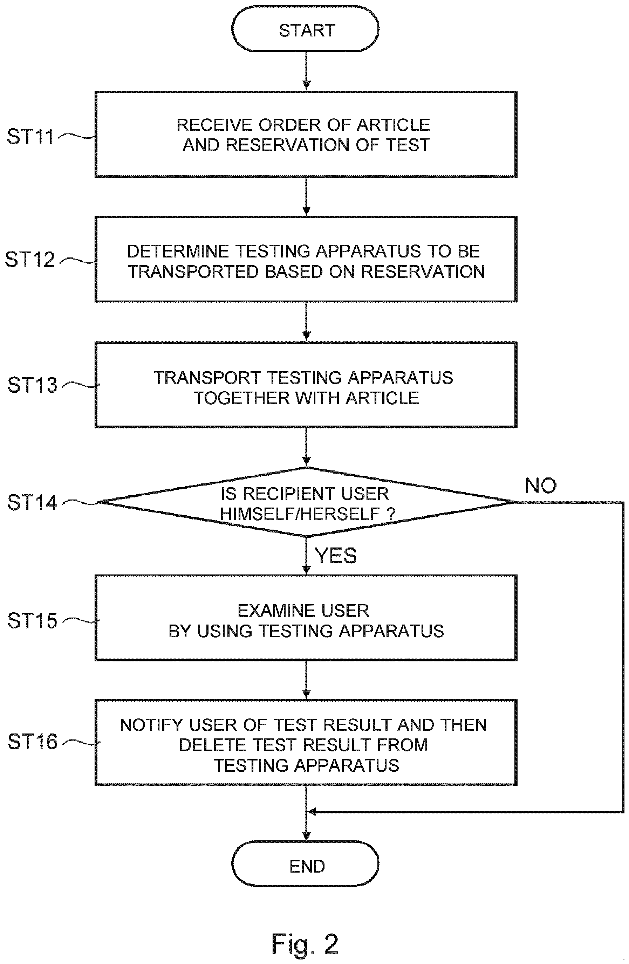

Next, a transportation management method according to the first embodiment will be described with reference to Fig. 2. Fig. 2 is a flowchart showing a transportation management method according to the first embodiment. In the following explanation using Fig. 2, Fig. 1 is also referred to as appropriate.

Firstly, as shown in Fig. 2, an order of an article(s) and a reservation for a test are received from a user (step ST11). For example, as shown in Fig. 1, the user enters order information oi into the user terminal 11. Note that the order information oi includes a reservation for a test that the user undergoes when the user receives the article.

Next, as shown in Fig. 2, a testing apparatus 50 to be transported is determined based on the reservation (step ST12). For example, a plurality of testing apparatuses are registered for each type of tests in the management server 20, and when a reservation for a test is included in the order information oi, a testing apparatus to be transported is determined from among the plurality of testing apparatuses based on the reservation.

More specifically, the management server 20 determines a type of a testing apparatus based on the type of the test reserved by the user. For example, when the test reserved by the user is an electrocardiographic test, an electrocardiographic testing apparatus is determined as the testing apparatus to be transported. Further, the management server 20 determines the testing apparatus to be transported based on information such as a desired delivery date and time, and a delivery destination (an address) included in the order information oi. For example, the management server 20 determines which of a plurality of electrocardiographic apparatuses registered in the management server 20 should be transported.

Next, as shown in Fig. 2, the testing apparatus 50 is transported by the transportation vehicle 40 together with the article (step ST13). Next, when the transportation vehicle 40 arrives at the delivery destination, it is confirmed whether or not the person who receives the article (hereinafter referred to as the recipient) is the user himself/herself who has reserved the test (step ST14). There are no particular restrictions on the method for authenticating the user. For example, the testing apparatus 50 may simply inquire of the recipient as to whether he/she is the user himself/herself and confirm it based on his/her answer. Alternatively, biometric authentication or the like may be used.

When the recipient of the article is the user himself/herself (YES in step ST14), the user is diagnosed by using the testing apparatus 50 (step ST15). On the other hand, when the recipient of the article is not the user himself/herself (NO in step ST14), the test does not need to be performed, so that the series of processes is finished.

Note that even when the recipient of the article is not the user himself/herself, the recipient may be asked whether or not to take (i.e., undergoes) the test, so that the recipient may take the test as in the case of the user himself/herself. On the other hand, even when the recipient of the article is the user himself/herself, the user may cancel the test on the spot.

Lastly, as shown in Fig. 2, the user is notified of the test result ir1 and then the test result ir1 is deleted from the testing apparatus 50 (step ST16). The test result ir1, which is personal information, can be prevented from being leaked.

Note that step ST16 is not indispensable.

As described above, in the transportation management method according to this embodiment, the testing apparatus 50 is transported to a user, and the user is diagnosed by using the testing apparatus 50. Therefore, in the transportation management method according to this embodiment, a user does not need to have a testing apparatus(es) in his/her house, so that a test can be carried out at a low cost. Further, in the transportation management method according to this embodiment, since the testing apparatus 50 is transported together with an article(s) ordered by the user, the test can be carried out at a lower cost than the cost that is required when only the testing apparatus 50 is transported.

### (Second Embodiment)

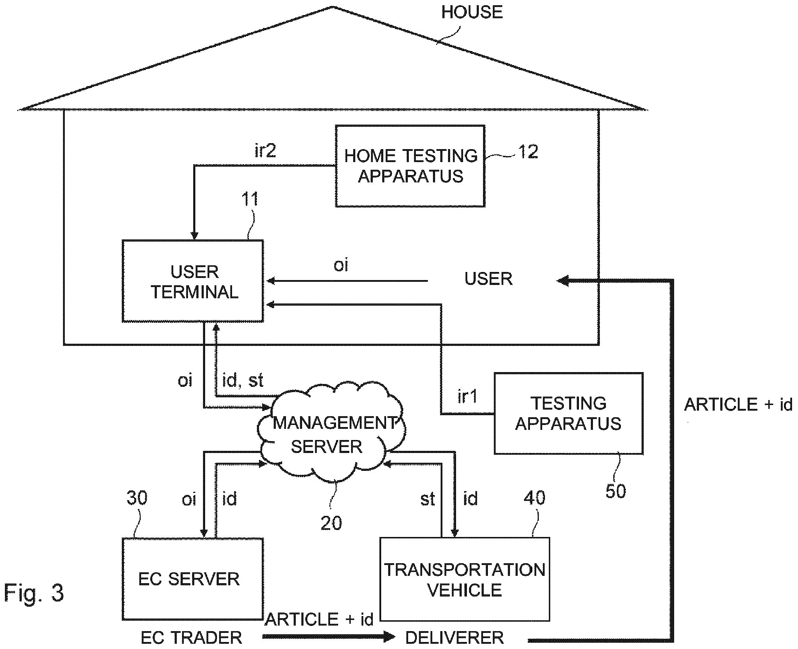

Next, a transportation management system and a transportation management method according to a second embodiment will be described with reference to Fig. 3. Fig. 3 is a block diagram of a transportation management system according to the second embodiment.

As shown in Fig. 3, the transportation management system according to this embodiment includes, in addition to the user terminal 11, the management server 20, the EC server 30, the transportation vehicle 40, and the testing apparatus 50 shown in Fig. 1, a home testing apparatus 12. The home testing apparatus 12 also includes, for example, an arithmetic unit and a storage unit in which various control programs, data, and the like are stored, functions as a computer, and performs the below-described processes based on the aforementioned various control programs.

The home testing apparatus 12 is a testing apparatus owned by a user and located in his/her house, and includes, for example, a thermometer, a scale (e.g., a bathroom scale), a sphygmomanometer, and the like. As shown in Fig. 3, the home testing apparatus 12 has a communication function, and a test result ir2 of the user obtained by the home testing apparatus 12 is transmitted to the user terminal 11. That is, as shown in Fig. 3, the user terminal 11 receives, in addition to the test result ir1 of the user obtained by the testing apparatus 50, the test result ir2 of the user obtained by the home testing apparatus 12.

Then, for example, the management server 20 may diagnose the health condition of the user based on the test results ir1 and ir2 received from the user terminal 11, and transmit the result of the diagnosis to the user terminal 11 (not shown). The result of the diagnosis may include a referral(s) to one or a plurality of hospitals. By referring the user to a hospital, the time and effort that the user takes to find a hospital can be reduced.

Alternatively, the test result ir2 may be transmitted from the home testing apparatus 12 to the testing apparatus 50 directly or through the user terminal 11, and the testing apparatus 50 may diagnose the health condition of the user based on the test result ir1 and ir2. Further, the home testing apparatus 12 may not have a communication function, and the user may enter the test result ir2 into the user terminal 11.

As described above, in the transportation management system according to this embodiment, since the health condition of the user is diagnosed based on, in addition to the test result ir1 of the testing apparatus 50, the test result ir2 of the home testing apparatus 12, the accuracy of the diagnosis is improved.

The rest of the configuration is similar to that of the first embodiment, and therefore the description thereof is omitted.

### (Third Embodiment)

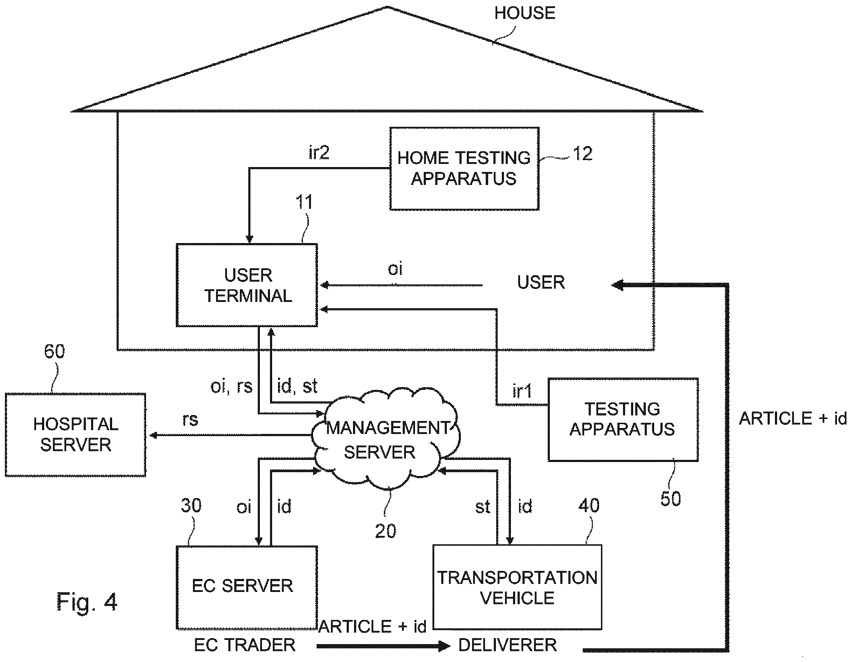

Next, a transportation management system and a transportation management method according to a third embodiment will be described with reference to Fig. 4. Fig. 4 is a block diagram of a transportation management system according to the third embodiment.

As shown in Fig. 4, the transportation management system according to this embodiment includes, in addition to the user terminal 11, the home testing apparatus 12, the management server 20, the EC server 30, the transportation vehicle 40, and the testing apparatus 50 shown in Fig. 3, a hospital server 60. The hospital server 60 also includes, for example, an arithmetic unit and a storage unit in which various control programs, data, and the like are stored, functions as a computer, and performs the below-described processes based on the aforementioned various control programs.

The hospital server 60 is, for example, a server constituting a website on the Internet managed by a hospital. As shown in Fig. 4, the hospital server 60 is connected to the management server 20 so as to be able to communicate with the management server 20, so that a user can make a reservation in the hospital. In the example shown in Fig. 4, when the user transmits reservation information rs from the user terminal 11, the hospital server 60 receives the transmitted reservation information rs through the management server 20.

The hospital server 60 may be a server of a hospital to which the user is referred through the user terminal 11. For example, the management server 20 refers the user to a hospital based on the test results ir1 and ir2. That is, the user can reserve the hospital to which he/she has been referred based on the test results ir1 and ir2.

Note that the management server 20 may refer the user to a hospital through, instead of the user terminal 11, the testing apparatus 50. Alternatively, the user may reserve a hospital from, instead of the user terminal 11, the testing apparatus 50. Further, the user may reserve the hospital based solely on the test result ir1 of the testing apparatus 50.

Further, the user may transmit the test results ir1 and ir2 together with the reservation information rs. The hospital, which has received the test results ir1 and ir2, can ask the user questions and/or give the user an advice through the management server 20 and the user terminal 11 before the user visits the hospital. For example, the hospital can advise the user as to whether the user should wait in his/her house or come to the hospital as soon as possible based on the test results ir1 and ir2 and the answers to the questions.

As described above, in the transportation management system according to this embodiment, the user is referred to a hospital based on the test result ir1 of the testing apparatus 50 and the test result ir2 of the home testing apparatus 12, and the user can make a reservation in the hospital to which he/she has been referred.

The rest of the configuration is similar to that of the second embodiment, and therefore the description thereof is omitted.

In the above-described examples, the various control programs can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g., magneto-optical disks), CD-ROM (Compact Disc Read Only Memory), CD-R (Compact Disc Recordable), CD-R/W (Compact Disc Rewritable), and semiconductor memories (such as mask ROM, PROM (Programmable ROM), EPROM (Erasable PROM), flash ROM, RAM (Random Access Memory), etc.). Further, the program may be provided to a computer using any type of transitory computer readable media. Examples of transitory computer readable media include electric signals, optical signals, and electromagnetic waves. Transitory computer readable media can provide the program to a computer through a wired communication line (e.g., electric wires, and optical fibers) or a wireless communication line.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A transportation management system configured to manage transportation of a testing apparatus (50) for diagnosing a health condition of a user by using a computer, the transportation management system being further configured to:
receive a reservation for a test that a user undergoes;
determine a testing apparatus (50) to be transported to the user based on the reservation; and
transport the testing apparatus (50) to the user and diagnose the user by using the transported testing apparatus (50).

2. The transportation management system according to Claim 1, wherein
the testing apparatus (50) is transported together with an article that the user has ordered, and
the user is diagnosed by using the testing apparatus (50) when the user receives the article.

3. The transportation management system according to Claim 1 or 2, wherein the testing apparatus (50) is put into and transported by an autonomous traveling vehicle.

4. The transportation management system according to any one of Claims 1 to 3, wherein after the user is diagnosed by using the testing apparatus (50), a result of the test of the user is deleted from the testing apparatus (50).

5. The transportation management system according to any one of Claims 1 to 4, wherein the user is referred to a hospital based on the result of the test obtained by diagnosing the user by using the testing apparatus (50).

6. A transportation management method for managing transportation of a testing apparatus (50) for diagnosing a health condition of a user by using a computer, the transportation management method comprising:
receiving a reservation for a test that a user undergoes;
determining a testing apparatus (50) to be transported to the user based on the reservation; and
transporting the testing apparatus (50) to the user and diagnosing the user by using the transported testing apparatus (50).

7. The transportation management method according to Claim 6, wherein
the testing apparatus (50) is transported together with an article that the user has ordered, and
the user is diagnosed by using the testing apparatus (50) when the user receives the article.

8. The transportation management method according to Claim 6 or 7, wherein the testing apparatus (50) is put into and transported by an autonomous traveling vehicle.

9. The transportation management method according to any one of Claims 6 to 8, wherein after the user is diagnosed by using the testing apparatus (50), a result of the test of the user is deleted from the testing apparatus (50).

10. The transportation management method according to any one of Claims 6 to 9, wherein the user is referred to a hospital based on the result of the test obtained by diagnosing the user by using the testing apparatus (50).

11. A program for managing transportation of a testing apparatus (50) for diagnosing a health condition of a user by using a computer, the program being adapted to cause the computer to:
receive a reservation for a test that a user undergoes;
determine a testing apparatus (50) to be transported to the user based on the reservation; and
transport the testing apparatus (50) to the user and diagnose the user by using the transported testing apparatus (50).
